# EUROPEAN PATENT APPLICATION

(11) **EP 2 866 033 A1**
(43) Date of publication of application: **29.04.2015**
(21) Application number: 13189926.2
(22) Date of filing: 23.10.2013
(51) Int. Cl.: G01N 33/68

(54) **Differential diagnosis of acute dyspnea based on C-terminal proSP-B, KL-6 and BNP-type peptides**

(71) Applicant: Roche Diagnostics GmbH, 68305 Mannheim (DE); F. Hoffmann-La Roche AG, 4070 Basel (CH)
(72) Inventor: Klemt, Volker, 82362 Weilheim (DE); Roeddiger, Ralf, 69517 Gorxheimertal (DE); Horsch, Andrea, 6006 Luzern (CH); Pfeffer, Michael, 82377 Penzberg (DE); Boehm, Christine, 6331 Huenenberg (CH); Ehret, Christoph, 81477 München (DE)
(74) Representative: Dick, Alexander

(57) **Abstract**

The present invention relates to a method for differentiating in a patient suffering from acute shortness of breath between various causes of said acute shortness of breath, said method comprising the steps of: measuring the level of C-terminal proSP-B or proSP-B in a sample of said patient, measuring the level of a BNP-type peptide in a sample of said subject, measuring the level of a KL-6 in a sample of said subject, and differentiating between the various causes by comparing the determined levels with reference levels. Further envisaged are kits and devices adapted to carry out the method of the present invention.

## Description

The present invention relates to a method for differentiating in a patient suffering from acute shortness of breath between various causes of said acute shortness of breath, said method comprising the steps of: measuring the level of C-terminal proSP-B or proSP-B in a sample of said patient, measuring the level of a BNP-type peptide in a sample of said subject, measuring the level of a KL-6 in a sample of said subject, and differentiating between the various causes by comparing the determined levels with reference levels. Further envisaged are kits and devices adapted to carry out the method of the present invention.

Dyspnea, the shortness of breath, is a symptom and is defined by the American Thoracic Society as a "subjective experience of breathing discomfort that consists of qualitatively distinct sensations that vary in intensity. The experience derives from interactions among multiple physiological, psychological, social, and environmental factors and may induce secondary physiological and behavioral responses (Parshall, MP et al. Am J Respir Crit Care Med Vol 185, Iss. 4, pp 435-452, Feb 15, 2012)

Frequently dyspnea is the consequence of deviations from normal function in the cardio-pulmonary systems and can include pulmonary, cardiovascular and mixed cardiopulmonary diseases for instance. Examples for non-cardio-pulmonary causes of dyspnea are neuromuscular diseases of the chest wall and anxiety.

Pulmonary diseases normally affect the mechanical properties of the respiratory system (airways, lung parenchyma, chest wall) and can lead to obstructions, restrictions of the airways and damages of the alveolar-capillary interface for instance. Hypoxemia, hypercapnia and a ventilation-perfusion mismatch can be the consequence. Pulmonary infections/pneumonia and chronic pulmonary diseases like asthma, COPD and interstitial lung diseases are typical lung diseases causing dyspnea.

Disorders of the cardiovascular system causing dyspnea are frequently resulting gas exchange abnormalities.

For instance an elevation of left-ventricular end-diastolic volume and pressure as well as increased pulmonary capillary pressures may be seen. In severe cases these elevated pressures may lead to interstitial edema in the lung. Diastolic dysfunction, characterized by a very stiff left ventricle, may lead to severe dyspnea as well. Cardiac diseases which may cause such malfunction include heart failure, valvular heart disease, cardiac arrhythmia.

Parshall et al. (see above) provides an overview of physiological causes resulting in dyspnea (see in particular table 5 in this document).

As dyspnea is a symptom it is difficult to quantify and to assess and given the broad differential diagnosis of dyspnea, a systematic approach in evaluation patients with dyspnea is required. Current medical practice for patients presenting with dyspnea comprises a combination of clinical findings, patient's medical history and results of various diagnostic methods like X-ray, echocardiography, lung function testing and laboratory testing.

As the above description may apply to a general hospital population, the symptom of dyspnea is especially difficult to assess and the differential diagnosis in a severely ill patient group. One example of such severely ill patients are patients with acute respiratory distress syndrome which are described further in the next paragraph.

The "gold standard" of determining the respiratory function in severe critical ill patients is the determination of the respiratory gas exchange by arterial blood-gas analysis which enables the direct measurement of pH, partial pressures of O₂ and CO₂, and O₂ saturation.

However, repetitive sampling of arterial blood is in critical ill patients is frequently difficult and painful that noninvasive monitoring of respiratory function is employed in the critical care setting instead. Noninvasive methods for monitoring the respiratory function include the pulse oximetry and the monitoring the mechanical ventilation. With pulse oximetry the arterial and venous O₂ saturation usually in a finger is determined.

The respiratory system function, i.e. the function of the lungs and/or chest wall can be monitored by the volume and pressure applied via the mechanical ventilation. Typically findings in patients with abnormal respiratory function include e.g. an elevated peak and plateau airway pressures but a normal gradient between peak and plateau airway pressures. Both methods are however indirect methods and do not allow the monitoring and differentiation of underlying pathophysiological conditions, i.e. the assessment of respiratory and cardiovascular function. For instance, the deterioration of the pulmonary function might be due to respiratory or/and the cardiovascular system but might not be to differentiate by either method.

Classical lung function testing is not possibly in those severely ill patients who are mechanically ventilated.

Pulmonary edema is defined as an accumulation of extravascular fluid in the lungs. Two major mechanisms may cause pulmonary edema. On the one hand it may develop as a consequence of movement of fluid into the interstitial tissue. This situation is frequently caused by a raised capillary hydrostatic pressure secondary to left-sided heart insufficiency or failure and is therefore named cardiogenic pulmonary edema. The heart is not able to sufficiently pump out the blood into the body and the blood back pressures into the lung. Initially this back pressure may become apparent as pulmonary congestion and eventually develop to pulmonary edema when the pressure further increases and fluid is pressed into the alveolar-capillary interstitium and the alveolar space. This fluid reduces the gas exchange of oxygen carbon dioxide and the patient experiences shortness of breath.

On the other hand pulmonary edema can develop following injuries of the alveolar-capillary barrier and is called non-cardiogenic pulmonary edema. In those cases the edema develops following the injury of the alveolar membrane and impairs gas diffusion and exchange. Pneumonia, acute respiratory distress syndrome and sepsis are typical diseases which can be associated with such non-cardiogeneic pulmonary edema and directly injure the alveolar capillary membrane.

The management of pulmonary edema comprises the treatment of the underlying cause and the improvement of the gas exchange; the latter ranging from supplemental oxygen supply to administration of mechanical ventilatory support depending on the severity of the disturbance in lung function.

Further, the differentiation of cardiogenic from non-cardiogenic pulmonary edema is essential for the treatment of the edema and congestion. The patient's medical history might be indicative for the cause in addition to a chest radiograph which is still a frequently applied method as it is quickly performed. In cardiogenic pulmonary edema typically an enlarged cardiac silhouette can be seen and cardiac redistribution might indicate the raised pressure and congestion. In contrast in noncardiogenic pulmonary edema the heart size is normal and the alveolar infiltrates might be more evenly distributed throughout the lungs. For more advanced differential diagnosis computer tomography and echocardiography of the heart as well as other diagnostic methods used in patients presenting with dyspnea like lung function testing, blood gas testing might be applied. Frequently, however, differential diagnosis of the dyspnea and pulmonary edema might be difficult to be achieved in severely ill patients when medical conditions are not allowing extensive differential diagnosis and in patients with pre-existing cardiac and pulmonary disease where it might be difficult to decide what caused the dyspnea and edema.

Patients suffering from acute heart insufficiency or heart failure are frequently treated with Angiotensin-converting enzyme (ACE) inhibitors and beta-blockers to treat the heart insufficiency. Mineralocorticoid receptor antagonist might be considered as well. The ACE inhibitors have a modest effect on remodeling of the particular left ventricular heart function and the beta-blockers are supposed to improve the cardiac ejection fraction. In patients with signs and symptoms of congestion and edema diuretics will be added to those medications in order to relieve dyspnea and edema. (ESC Guideline 2012, European Journal of Heart Failure (2012) 14, 803-869).

In patients with ARDS and ALI, as one example of patients with noncardiogenic pulmonary edema, fluid restriction and diuretics is also applied to restrict pulmonary edema and to maintain cardiac function. Therapy in those patients is also targeting the underlying disorders like for instance pneumonia, sepsis, or trauma and the management of the ventilation by mechanical support. (Harrison's Internal Medicine Online, Part 12, Critical care Medicine, Chapter 268 Acute Respiratory Distress Syndrome)

WO99/13337 discloses that surfactant proteins may be used as a biomarker for several specific pulmonary diseases including acute respiratory distress syndrome (ARDS).

In WO2004/077056 it is disclosed that systemic levels of surfactant proteins may be used as markers for heart failure. However, the disclosed techniques do not allow for a differential diagnosis of the cause of the elevated levels of the surfactant proteins. Specifically, it is known that pulmonary diseases or damages may also result in increased systemic levels of said proteins (Doyle 1997, Am J Respir Crit Care Med Vol. 156: 1217-1229). Accordingly, the disclosed methods shall inevitably produce false positive diagnostic results which in turn result in an inappropriate therapy (Svendstrup Nielsen, 2004, The European Journal of Heart Failure 6: 63-70 ).

Moreover, surfactant proteins have been described together with the N-terminal brain natriuretic Peptide (NT-proBNP) to be indicative for the severeness of heart failure according to the New York Hear Association (NYHA) classification (DePasquale, 2004, Circulation 110:1091-1096).

NT-proBNP has been also described together with seven additional parameters as an indicator for acute heart failure in patients exhibiting dyspnea (Baggish 2005, American Heart Journal, 151:48-54).

Krebs von den lungen-6 (KL-6) is a mucin-like glycoprotein (MUC1 mucin) expressed at the extracellular surface of alveolar type II cells and bronchiolar epithelial cells. It acts as a chemotactic factor that promotes migration, proliferation, and survival of lung fibroblasts. It has been studied in many interstitial lung diseases as a nonspecific marker of fibrosis. Serum KL-6 levels are significantly elevated in patients with IPF compared to healthy volunteers (see Vij R., Translational Research 2012;159:218-227).

Ichiyasu et al. investigated the correlation of KL-6 and surfactant protein D serum levels with high resolution CT (HRCT) findings in patients diagnosed of nonspecific interstitial pneumonia (NSIP)and which are considered to be common in nonspecific interstitial pneumonia like of ground-glass attenuation, airspace consolidation, traction bronchiectasis, and honeycombing, and their different combinations. The investigators had developed a score for the presence of each those findings in the HRCT and tested the association between the HRCT score and the biomarker serum levels.

Statistical analysis revealed a significant correlation between the HRCT score and the KL-6 levels while no correlation was observed between the HRCT score and the SP-D levels in a group of 21 patients with NSIP. The serum level of SP-D was yet positively correlated with the extent of ground-glass attenuation without traction bronchiectasis and the inflammatory pattern. (Ichiyasu H et al. Respiration. 2012;83(3):190-7).

Kl-6 was further described to be elevated in epithelial lining fluid and plasma specimens and patients with ARDS. Serial plasma KL-6 concentrations were higher in non-survivors compared to survivors. (Ishizaka A. et al. Am J Physiol Lung Cell Mol Physiol. 2004 Jun;286(6):L 1088-94)

Kobayashi et al. found differences in the immunolocalization of surfactant protein A and D and KL-6 in patients with pulmonary alveolar proteinosis, a rare congenital or idiopathic lung diseases. They found that KL-6 was distributed in the alveolar epithelial cells and in the intra-alveolar substance. SP-A was similarly distributed as KL-6 while SP-D was mainly found in the SP-A-negative foci. SP-B or SP-C were not investigated in this study (Kobayashi M et al. Pathol Int. 2008 Mar;58(3):203-7).

However, Brasch et al. investigated the surfactant protein distribution in adult patients with pulmonary alveolar proteinosis by immunohistochemistry and Western blotting and also looked at the distribution of the precursor forms of surfactant protein B. In the diseased patients the precursor forms of SP-B were mainly found intra-alveolarly and in the alveolar proteinosis fluids and only very weakly in the cytoplasma of type-II pneumocytes. (Eur Respir J. 2004 Sep;24(3):426-35)

EP 1 882 945 Al discloses a method for differentiating in a patient suffering from acute shortness of breath between various causes of shortness of breath. The document, however, does not disclose the diagnosis of pulmonary edema and congestion.

Therefore, there is a clear long-standing need for means and methods allowing a differential diagnosis of the cause of symptoms such as acute dyspnea in a subject. The said means and methods shall allow a reliable efficient diagnosis and shall avoid the drawbacks of the current techniques.

Accordingly, the present invention relates to a method for differentiating in a patient suffering from acute shortness of breath between
(a) a pulmonary disease with (a1) pulmonary edema or without (a2) pulmonary edema,
(b) a cardiovascular complication with (b1) pulmonary edema and/or congestion, or without (b2) pulmonary edema and/or (in particular "and") congestion,
(c) a cardiovascular complication accompanied by a pulmonary disease with (c1) pulmonary edema and/or congestion, or without (c2) pulmonary edema and/or (in particular "and") congestion, and/or
(d) acute dyspnea without cardiovascular or pulmonary causes,
   said method comprising the steps of:
   i) measuring the level of C-terminal proSP-B or proSP-B in a sample of said patient,
   ii) measuring the level of a BNP-type peptide in a sample of said subject,
   iii) measuring the level of a KL-6 in a sample of said subject, and
   iv) differentiating between (a1), (a2), (b1), (b2), (c1), (c2) and (d) by comparing the levels determined in i), ii) and iii) with reference levels.

In an embodiment, the level of the biomarkers as referred to under i), ii), and iii) is measured by contacting the sample with an agent that specifically binds to the respective biomarker, thereby forming a complex between the agent and said marker, detecting the amount of complex formed, and thereby measuring the level of said biomarker.

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (i), (ii), (iii) and/or (iv) may in total or in part be assisted by automation, e.g., by a suitable robotic and sensory equipment for the measurement in steps (i), (ii), or (iii) or a computer-implemented comparison and/or assessment based on said comparison in step (iv).

The "patient" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the patient is a human patient. The terms "subject" and "patient" are used interchangeably herein. In an embodiment, it is envisaged that the patient does not suffer from cancer.

In an embodiment, the patient requires mechanical ventilation. Mechanical ventilation is a method to mechanically assist or replace spontaneous breathing. Mechanical ventilation is indicated when the patient's spontaneous ventilation is inadequate to maintain life. In an embodiment, it is thus envisaged that the patient is a severely ill patient. Various mechanical ventilatory strategies such as low tidal volume ventilation, open lung ventilation, and high positive end-expiratory pressure may be applied.

In an embodiment, the patient also requires intensive care.

Further, is it envisaged that the patient may suffer from ARDS (Acute respiratory distress syndrome). Acute respiratory distress syndrome (ARDS), also known as respiratory distress syndrome (RDS) or adult respiratory distress syndrome is a severe, life-threatening reaction in adult humans to injuries or acute infections to the lung. It is a lung syndrome that causes inflammation of the lung parenchyma leading to impaired gas exchange with a systemic release of inflammatory mediators, causing inflammation, hypoxemia and frequently multiple organ failure. The onset can be within minutes and few hours of the injury. A less severe form is called acute lung injury (ALI). The diagnostic criteria for ARDS are based on the oxygenation status and are expressed by the ratio of arterial O₂ partial pressure versus inspiratory O₂ fraction. The arterial (a) PO₂ (in mmHg)/FIO₂ (inspiratory O₂ fraction) <200 mmHg is characteristic of ARDS, while a Pa_{O2}/ F_{IO2} between 200 and 300 identifies patients with ALI.

The risk of developing ARDS is increasing in patients suffering from more than one predisposing medical or surgical condition. The risk for ARDS increases from 25% in patients with severe trauma to 56% in patients with trauma and sepsis. Trauma patients with an acute physiology and chronic health evaluation (APACHE) II score ≥16 have a 2.5-fold increase in the risk of developing ARDS, and those with a score >20 have an incidence of ARDS that is more than threefold greater than those with APACHE II scores 9.

In accordance with the present invention, the patient shall suffer from acute shortness of breath (dyspnea). The expression "acute shortness of breath" or "acute dyspnea" is well known by the skilled person. As used herein, the expression refers to an impaired respiration which results in an increased respiratory frequency and/or an increased respiratory volume. Thus, shortness of breath may result, preferably, in hyperventilation. Shortness of breath occurs, usually, at an oxygen saturation level below the normal oxygen saturation level of at least 95%. Acute dyspnea as used herein refers to a non-permanently occurring shortness of breath, i. e. shortness of breath which occurs all over sudden (acute onset dyspnea) and which is not correlated to a specific condition, e. g., which occurs only under certain types of stress etc.. Moreover, acute dyspnea persists no longer than 2 weeks from the acute onset, while chronic dyspnea is characterized as persisting for a period of time longer than 2 weeks. Furthermore, acute dyspnea is, usually, progressively worsening.

The term "pulmonary disease", preferably, refers to any pulmonary disease which causes shortness of breath. Moreover, it is envisaged that a pulmonary disease as referred to in accordance with the present invention shall result in an impaired alveolocapillary membrane barrier having an increased permeability for surfactant proteins, in particular for the pulmonary surfactant protein specifically referred to herein. Said disease is, preferably, acute and chronic respiratory failure, COPD, pulmonary hypertonia, pneumonia, pulmonary fibrosis, pulmonary proteinosis, pulmonary inflammation, pulmonary emphysema obesity, thyroid diseases or, more preferably, a pulmonary embolism.

The term "cardiovascular complication" as used herein refers to any acute or chronic disorder of the cardiovascular system. Acute disorders of the cardiovascular system include acute cardiovascular events. Thus, more preferably, encompassed are stable angina pectoris (SAP) or acute coronary syndromes (ACS). ACS patients can show unstable angina pectoris (UAP) or these individuals have already suffered from a myocardial infarction (MI). MI can be an ST-elevated MI or a non-ST-elevated MI. The occurring of an MI can be followed by a left ventricular dysfunction (LVD). Also encompassed by the term are chronic disorders and, preferably, heart failure. It is to be understood that the term also includes medical conditions and diseases which cause heart failure in addition to the aforementioned acute cardiovascular events, such as congenital or acquired heart valve diseases or disorders, myocarditis, myocardiopathy, amyloidosis or hemochromatosis. Further preferred cardiovascular diseases are thrombosis, preferably arterial thrombosis, or diseases causing blood vessel calcification, preferably atherosclerosis, as well as stroke.

Preferably, the cardiovascular complication is heart failure.

The term "heart failure" as used herein relates to an impaired systolic and/or diastolic function of the heart being accompanied by overt signs of heart failure as known to the person skilled in the art. Preferably, heart failure referred to herein is also chronic heart failure. Heart failure according to the present invention includes overt and/or advanced heart failure. In overt heart failure, the patient shows symptoms of heart failure as known to the person skilled in the art.

HF can be classified into various degrees of severity.

According to the NYHA (New York Heart Association) classification, heart failure patients are classified as belonging to NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (ACC/AHA classification, see J. Am. Coll. Cardiol. 2001;38;2101-2113, updated in 2005, see J. Am. Coll. Cardiol. 2005;46;e1-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", in particular, refers to stages B, C and D of the ACC/AHA classification referred to above. In these stages, the patient shows typical symptoms of heart failure and/or shows structural and/or functional abnormalities of the heart. Accordingly, a patient who suffers from heart failure, suffers from heart failure classified as stage B, C or D ac-cording to the ACC/AHA classification. Also preferably, the heart failure may be classified as NYHA class II; III or IV. In a preferred embodiment, the term "heart failure" refers to stages B and C of the ACC/AHA classification referred to above, or to heart failure classified as NYHA class II or class III. Accordingly, the patient may suffer from heart failure classified as stage B or C according to the ACC/AHA classification. Also preferably, the patient may suffer from heart failure classified as NYHA class II or III.

By "a cardiovascular complication accompanied by a pulmonary disease" it is meant that the patient shall suffer from a cardiovascular complication and from a pulmonary disease. It is to be understood that the two diseases or disorders may appear independently, i.e. without one causing the other. However, cases in which a primary cardiovascular complication as referred to herein causes a secondary pulmonary diseases and vice versa are also, preferably, encompassed from the above expression.

Further, acute dyspnea may be observed in patients which neither suffer from cardiovascular complications nor from pulmonary diseases. Such cases shall be comprised by the term "acute dyspnea without cardiovascular or pulmonary causes" for the purpose of the present invention. Preferred non-cardiovascular and non-pulmonary causes of acute shortness of breath are, preferably, obesity, high body weight, an untrained or poorly trained physical condition of the subject, psychological conditions such as anxiety states.

The pulmonary disease under (a) may be accompanied by pulmonary edema (a1) or not (a2). The cardiovascular complication under (b) may be accompanied by pulmonary edema and/or congestion (b1), or may not be accompanied by pulmonary edema and/or congestion (b2). Further, the cardiovascular complication accompanied by pulmonary disease under (c) may be accompanied by pulmonary edema and/or congestion (c1), or may not be accompanied by pulmonary edema and/or congestion (c2).

The terms "pulmonary edema" and "congestion" are well know in the art.

The term "congestion" as used herein preferably refers to pulmonary congestion. Pulmonary congestion occurs when the heart is no longer able to adequately support the respiratory system. It develops as fluid backs up into the lungs. As the heart fails to pump sufficient blood through or coming from the lungs, blood pressure within the lungs increases causing fluid to accumulate in the alveoli. Thus, as set forth above herein below in connection with cardiogenic pulmonary edema, the blood back pressures into the lung. Pulmonary congestion may lead to pulmonary edema if the fluid leaks into the tissue. Accordingly, pulmonary congestion may precede pulmonary edema (cardiogenic pulmonary edema, see also next paragraph). Thus, a patient may have pulmonary congestion alone, or both pulmonary congestion and pulmonary edema (in particular, cardiogenic pulmonary edema).

Pulmonary edema is defined as an accumulation of extravascular fluid in the lungs. Two major mechanisms may cause pulmonary edema. On the one hand it may develop as a consequence of movement of fluid into the interstitial tissue. This situation is preferably caused by a raised capillary hydrostatic pressure secondary to left-sided heart insufficiency or failure and is therefore named cardiogenic pulmonary edema. The heart is not able to sufficiently pump out the blood into the body and the blood back pressures into the lung. Initially this back pressure may become apparent as pulmonary congestion and eventually develop to pulmonary edema when the pressure further increases and fluid is pressed into the alveolar-capillary interstitium and the alveolar space. This fluid reduces the gas exchange of oxygen carbon dioxide and the patient experiences shortness of breath.

On the other hand pulmonary edema can develop following injuries of the alveolar-capillary barrier and is called non-cardiogenic pulmonary edema. In those cases the edema develops following the injury of the alveolar membrane and impairs gas diffusion and exchange. Pneumonia, acute respiratory distress syndrome and sepsis are typical diseases which can be associated with such non-cardiogenic pulmonary edema and directly injury the alveolar capillary membrane.

Accordingly, the pulmonary edema is preferably non-cardiogenic pulmonary edema if it is caused by pulmonary disease and cardiogenic pulmonary edema if it is caused by a cardiovascular complication. Accordingly, the pulmonary edema as set forth under (a) of the method of the present invention is preferably non-cardiogenic pulmonary edema. Further, the pulmonary edema as set forth under (b) of the method of the present invention is preferably cardiogenic pulmonary edema. In addition, the pulmonary edema as set forth under (c) may be non-cardiogenic pulmonary edema, or cardiogenic pulmonary edema, or both.

Thus, it is envisaged to differentiate between
(a) a pulmonary disease with (a1) non-cardiogenic pulmonary edema or without (a2) pulmonary edema,
(b) a cardiovascular complication with (b1) cardiogenic pulmonary edema and/or congestion, or without (b2) pulmonary edema and/or congestion,
(c) a cardiovascular complication accompanied by a pulmonary disease with (c1) cardiogenic or non-cardiogenic pulmonary edema and/or congestion, or without (c2) pulmonary edema and/or congestion, and
(d) acute dyspnea without cardiovascular or pulmonary causes.

By carrying out the aforementioned method, it can be differentiated between various causes of acute shortness of breath as set forth under (a1), (a2), (b1), (b2), (c1), (c2) and (d) of the method of the present invention. The term "differentiating" as used herein means to distinguish between a patient which suffers from (a1), (a2), (b1), (b2), (c1), (c2), or (d) as set forth in the method of the present invention. The term as used herein, preferably, includes differentially diagnosing (a1), (a2), (b1), (b2), (c1), (c2), or (d).

Diagnosing as used herein refers to assessing the probability according to which a patient suffers from the diseases referred to in this specification. As will be understood by those skilled in the art, such an assessment is usually not intended to be correct for 100% of the subjects to be diagnosed. The term, however, requires that a statistically significant portion of subjects can be diagnosed to suffer from the said disease (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983 . Preferred confidence intervals are at least 90%, at least 95%, at least 97%, at least 98% or at least 99 %. The p-values are, preferably, 0.1, 0.05, 0.01, 0.005, or 0.0001.

The term "sample" refers to a sample of a body fluid, to a sample of separated cells or to a sample from a tissue or an organ. Samples of body fluids can be obtained by well known techniques and include, preferably, samples of blood, plasma, serum, or urine. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Preferred samples are blood, plasma, or serum samples.

The "sample" can obtained from the patient at any time after the onset of acute shortness of breath. In an embodiment, the sample is obtained at presentation at a physician or at the emergency room or up to ten days after presentation. However, it is envisaged that the patient still suffers from acute shortness of breath when the sample is obtained.

In an embodiment, it is envisaged that the sample is obtained within the first 24 hours after the onset of acute shortness of breath. In another embodiment, it is envisaged that the sample is obtained on the second day or the third day after the onset. However, it is also contemplated that the sample is obtained later such as on the 5^{th} day or the 7^{th} day after the onset.

The level of the biomarkers as referred to herein can be determined in the same sample or in different samples from the patient.

The term "measuring" the level of a biomarker as referred to herein, C-terminal pro-SP-B or pro-SP-B, a BNP-type peptide and KL-6, refers to the quantification of the biomarker, e.g. to determining the level of the biomarker in the sample, employing appropriate methods of detection described elsewhere herein.

The biomarkers C-terminal proSP-B and proSP-B are well known in the art and e.g. are disclosed in WO 2012/130731 which herewith is incorporated by reference with respect to the entire disclosure content, in particular with respect to the biomarkers C-terminal proSP-B and proSP-B as well as the sequences thereof. Also the sequence listing is incorporated by reference. Preferably, the definition for C-terminal proSP-B and proSP-B given in WO 2012/130731 also applies to the present invention.

Surfactant Protein B (SP-B) is a 79-amino acid peptide, produced by the proteolytic cleavage of SP-B proprotein (proSP-B; for a sequence of the biomarker proSP-B, see e.g. SEQ ID NO: 1 of WO 2012/130731) that is processed to a smaller, lipid-associated peptide in the distal secretory pathway within the type II cell. Processing of proSP-B occurs in the multivesicular body (MVB) and lamellar body (LB) compartments.

SP-B facilitates the stability and rapid spreading of surfactant phospholipids during respiratory cycles. It maintains the molecular continuity of the monolayer of the lipid and peptide at the air-water interface during breathing and facilitates the incorporation of lipid from the lung aqueous subphase into the lipid monolayer at the alveolar air-water interface. Processing of the SP-B preproprotein to its mature peptide occurs during transit through the secretory pathway from the endoplasmic reticulum to the Golgi network and the multivesicular bodies in the type II epithelial cell.

Entry of the SP-B preproprotein into the secretory pathway is mediated by the N-terminal signal peptide which is cleaved upon translation of the proprotein into the endoplasmic reticulum. Transit of SP-B out of the endoplasmic reticulum is dependent on the N-terminal propeptide, which likely facilitates folding and/or sequestration of the hydrophobic mature peptide. The C-terminal propeptide, (amino acids 280 to 381 of proSP-B = SEQ ID NO: 2 of WO 2012/130731) however, seems not to be required for sorting of SP-B to secretory granules.

C-terminal proSP-B in the sense of the present invention, preferably, relates to proSP-B and all cleavage products or fragments thereof comprising the C-terminal sequence of proSP-B. Thus, the measurement of the level of C-terminal proSP-B may encompass the measurement of any polypeptide which comprises said C-terminal sequence. In an embodiment, the C-terminal sequence encompasses about 100 amino acids, in particular 101 amino acids at the C-terminus of proSP-B. In particular, the C-terminal sequence may span from amino acids 280 to 381 of proSP-B (see SEQ ID NO: 2 in WO 2012/130731 which shows these 101 amino acids). In another embodiment, the C-terminal sequence may span from amino acids 285 to 334 of proSP-B (for this region see SEQ ID NO: 3 in WO 2012/130731).

It is preferred that the C-terminal sequence is the sequence as defined in SEQ ID NO: 3 of WO 2012/130731. Accordingly, the measurement of C-terminal proSP-B thus relates in particular to the measurement of proSP-B and those fragments or cleavage products thereof comprising SEQ ID NO:3 as disclosed WO 2012/130731. In the present invention C-terminal proSP-B thus may encompass proSP-B and those proSP-B fragments comprising the C-terminal sequence of proSP-B as defined in SEQ ID NO:3 of WO 2012/130731.

C-terminal proSP-B may include but is not limited to the following: proSP-B (i.e. the proSP-B of SEQ ID NO:1 of WO 2012/130731, comprising the N-terminal propeptide sequence the sequence stretch representing the mature SP-B and the C-terminal pro SP-B); the mid-molecular plus C-terminal fragment (i.e. the amino acids from position 201 to 381 of SEQ ID NO:1 of WO 2012/130731) and the C-terminal proSP-B fragment (i.e. the amino acids from position 280 to 381 of SEQ ID NO:1 of WO 2012/130731). As obvious to the artisan, proSP-B and fragments comprising the C-terminal proSP-B sequence of SEQ ID NO: 3 of WO 2012/130731 may be present as monomers and/or dimers, respectively.

For determination of C-terminal proSP-B the sample obtained from an individual is incubated in vitro with the specific binding agent for C-terminal proSP-B under conditions appropriate for formation of a binding agent C-terminal proSP-B complex. Such conditions need not be specified, since the skilled artisan without any inventive effort can easily identify such appropriate incubation conditions. As the skilled artisan will appreciate there are numerous methods to determine the level of the specific binding agent C-terminal proSP-B complex all described in detail in relevant textbooks (cf., e.g., Tijssen, P., supra, or Diamandis, E.P., and Christopoulos, T.K. (eds.), Immunoassay, Academic Press, Boston (1996)).

Assays for the determination of the level of proSP-B and C-terminal proSP-B are disclosed in Examples 1 and 2, respectively, of WO 2012/130731 which are incorporated by reference as well.

As used herein, the term "BNP-type peptides" comprise pre-proBNP, proBNP, NT-proBNP, and BNP. The pre-pro peptide (134 amino acids in the case ofpre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, BNP-type peptides according to the present invention are NT-proBNP, BNP, and variants thereof. BNP is the active hormone and has a shorter half-life than the respective inactive counterpart NT-proBNP. BNP is metabolized in the blood, whereas NT-proBNP circulates in the blood as an intact molecule and as such is eliminated renally. The in-vivo half-life of NT-proBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measure-ment of the active or the inactive forms of the natriuretic peptide can be advantageous. The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corre-sponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level preferably, at least 50%, 60%, 70%, 80%, 85%, 90%, 92%, 95%, 97%, 98%, or 99% identical to human NT-proBNP, preferably over the entire length of human NT-proBNP. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of human NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Lab Invest 230:177-181), Yeo et al. (Yeo 2003, Clinica Chimica Acta 338:107-115). Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label, to the peptide.

Krebs von den lungen-6 (KL-6) is a mucin-like glycoprotein (MUC1 mucin) expressed at the extracellular surface of alveolar type II cells and bronchiolar epithelial cells. It acts as a chemotactic factor that promotes migration, proliferation, and survival of lung fibroblasts. It has been studied in many interstitial lung diseases as a nonspecific marker of fibrosis. Serum KL-6 levels are significantly elevated in patients with IPF compared to healthy volunteers (see Vij R., Translational Research 2012;159:218-227).

In an embodiment, the levels biomarkers are measured as described in the examples.

In an embodiment, the biomarkers to be measured are proteins.

The biomarkers as referred to herein can be detected using methods generally known in the art. Methods of detection generally encompass methods to quantify the level of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence-based immunoassays, which are commercially available. Further suitable methods to detect biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass- spectrometers, NMR- analyzers, or chromatography devices. Further, methods include microplate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used immunoassays.

Methods for measuring electrochemiluminescent phenomena are well-known. Such methods make use of the ability of special metal complexes to achieve, by means of oxidation, an excited state from which they decay to ground state, emitting electrochemiluminescence. For review see Richter, M.M., Chem. Rev. 104 (2004) 3003-3036.

Biomarkers can also be detected by generally known methods including magnetic resonance spectroscopy (NMR spectroscopy), Gas chromatography-mass spectrometry (GC-MS), Liquid chromatography-mass spectrometry (LC-MS), High and ultra-HPLC HPLC such as reverse phase HPLC, for example, ion-pairing HPLC with dual UV-wavelength detection, capillary electrophoresis with laser-induced fluorescence detection, anion exchange chromatography and fluorescent detection, thin layer chromatography.

Preferably, measuring the level of a biomarker as referred to herein comprises the steps of (a) contacting a cell capable of eliciting a cellular response the intensity of which is indicative of the level of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the level of the peptide or polypeptide.

Also preferably, measuring the level of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Measuring the level of a peptide or polypeptide may, preferably, comprises the steps of (a) contacting the peptide with a specific binding agent, (b) (optionally) removing non-bound binding agent, (c) measuring the level of bound binding agent, i.e. the complex of the binding agent formed in step(a). According to a preferred embodiment, said steps of contacting, removing and measuring may be performed by an analyzer unit of the system disclosed herein. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound binding agent, i.e. the binding agent or the binding agent/peptide complex, will generate an intensity signal. Binding according to the present invention includes both covalent and non-covalent binding. A binding agent according to the present invention can be any compound, e.g., a peptide, polypeptide, nucleic acid, or small molecule, binding to the peptide or polypeptide described herein. Preferred binding agents include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such binding agents are well-known in the art. For example, identification and production of suitable antibodies or aptamers is also offered by commercial suppliers. The person skilled in the art is familiar with methods to develop derivatives of such binding agents with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the binding agent or agent binds specifically to the pep-tide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the binding agent can be measured by any method known in the art. Preferably, said method is semi-quantitative or quantitative. Further suitable techniques for the determination of a polypeptide or peptide are described in the following.

Binding of a binding agent may be measured directly, e.g. by NMR or surface plasmon resonance. Measurement of the binding of a binding agent, according to preferred embodiments, is performed by an analyzer unit of a system disclosed herein. Thereafter, an level of the measured binding may be calculated by a computing device of a system disclosed herein. If the binding agent also serves as a substrate of an enzymatic activity of the peptide or polypeptide of interest, an enzymatic reaction product may be measured (e.g. the level of a protease can be measured by measuring the level of cleaved substrate, e.g. on a Western Blot). Alternatively, the binding agent may exhibit enzymatic properties itself and the "binding agent/peptide or polypeptide" complex or the binding agent which was bound by the peptide or polypeptide, respectively, may be contacted with a suitable substrate allowing detection by the generation of an intensity signal. For measurement of enzymatic reaction products, preferably the level of substrate is saturating. The substrate may also be labeled with a detectable label prior to the reaction. Preferably, the sample is contacted with the substrate for an adequate period of time. An adequate period of time refers to the time necessary for a detectable, preferably measurable, level of product to be produced. Instead of measuring the level of product, the time necessary for appearance of a given (e.g. detectable) level of product can be measured. Third, the binding agent may be coupled covalently or non-covalently to a label allowing detection and measurement of the binding agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of tertiary binding agent binding to the secondary binding agent. The use of secondary, tertiary or even higher order binding agents is often used to increase the signal. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus hae-magglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluo-rescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, avail-able as ready-made stock solution from Roche Diagnostics), CDP-Star™ (Amersham Bio-sciences), ECF™ (Amersham Biosciences). A suitable enzyme-substrate combination may result in a colored reaction product, fluorescence or chemoluminescence, which can be measured according to methods known in the art (e.g. using a light-sensitive film or a suit-able camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The level of a peptide or polypeptide may be, also preferably, determined as follows: (a) contacting a solid support comprising a binding agent for the peptide or polypeptide as specified above with a sample comprising the peptide or polypeptide and (b) measuring the level peptide or polypeptide which is bound to the support. The binding agent, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The binding agent or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said binding agent are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different binding agents. Methods of producing such arrays, for example based on solid-phase chemistry and photo-labile protective groups, are generally known (US 5,744,305).

In an embodiment of the method of the present invention, the levels of the biomarkers as referred to herein are measured by using the assays described in the Examples section.

In another embodiment of the method of the present invention, the measurement in step a) may be carried out by an analyzer unit, in particular by an analyzer unit as defined elsewhere herein.

The term "binding agent" refers to a molecule that comprises a binding moiety which specifically binds the corresponding to the respective biomarker.

The term "specific binding" or "specifically bind" refers to a binding reaction wherein binding pair molecules exhibit a binding to each other under conditions where they do not significantly bind to other molecules. The term "specific binding" or "specifically binds", when referring to a protein or peptide as biomarker, refers to a binding reaction wherein a binding agent binds to the corresponding biomarker with an affinity of at least 10⁻⁷ M. The term "specific binding" or "specifically binds" preferably refers to an affinity of at least 10⁻⁸ M or even more preferred of at least 10⁻⁹ M for its target molecule. The term "specific" or "specifically" is used to indicate that other molecules present in the sample do not significantly bind to the binding agent specific for the target molecule. Preferably, the level of binding to a molecule other than the target molecule results in a binding affinity which is only 10% or less, more preferably only 5% or less of the affinity to the target molecule.

Examples of "binding agents" are a nucleic acid probe, nucleic acid primer, DNA molecule, RNA molecule, aptamer, antibody, antibody fragment, peptide, peptide nucleic acid (PNA) or chemical compound. A preferred binding agent is an antibody which specifically binds to the biomarker to be measured. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. Preferably, the antibody is a polyclonal antibody. More preferably, the antibody is a monoclonal antibody.

Another binding agent that can be applied, in an aspect, may be an aptamere which specifically binds to the at least one marker in the sample. The term "specific binding" or "specifically binds", when referring to a nucleic acid aptamer as a binding agent, refers to a binding reaction wherein a nucleic acid aptamer binds to the corresponding target molecule with an affinity in the low nM to pM range.

In yet an aspect the, sample is removed from the complex formed between the binding agent and the at least one marker prior to the measurement of the level of formed complex. Accordingly, in an aspect, the binding agent may be immobilized on a solid support. In yet an aspect, the sample can be removed from the formed complex on the solid support by applying a washing solution. The formed complex shall be proportional to the level of the at least one marker present in the sample. It will be understood that the specificity and/or sensitivity of the binding agent to be applied defines the degree of proportion of at least one marker comprised in the sample which is capable of being specifically bound. Further details on how the determination can be carried out are also found elsewhere herein. The level of formed complex shall be transformed into an level of at least one marker reflecting the level indeed present in the sample. Such an level, in an aspect, may be essentially the amount present in the sample or may be, in another aspect, an amount which is a certain proportion thereof due to the relationship between the formed complex and the amount present in the original sample.

The term "level" as used herein encompasses the absolute amount of a biomarker as referred to herein, the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein refers to comparing the level of the biomarkers in the sample from the individual or patient with the reference level of the biomarkers specified elsewhere in this description. It is to be understood that comparing as used herein usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from a reference sample. The comparison may be carried out manually or computer assisted. Thus, the comparison may be carried out by a computing device (e.g., of a system disclosed herein). The value of the measured or detected level of the biomarker in the sample from the individual or patient and the reference level can be, e.g., compared to each other and the said comparison can be automatically carried out by a computer program executing an algorithm for the comparison. The computer program carrying out the said evaluation will provide the desired assessment in a suitable output format. For a computer assisted comparison, the value of the determined amount may be compared to values corresponding to suitable references which are stored in a database by a computer program. The computer program may further evaluate the result of the comparison, i.e. automatically provide the desired assessment in a suitable output format. For a computer assisted comparison, the value of the determined amount may be compared to values corresponding to suitable references which are stored in a database by a computer program. The computer program may further evaluate the result of the comparison, i.e. automatically provides the desired assessment in a suitable output format.

In certain embodiments, the term "reference level" herein refers to a predetermined value for the respective biomarker. In this context "level" encompasses the absolute amount, the relative amount or concentration as well as any value or parameter which correlates thereto or can be derived therefrom. As the skilled artisan will appreciate the reference level is predetermined and set to meet routine requirements in terms of e.g. specificity and/or sensitivity. These requirements can vary, e.g. from regulatory body to regulatory body. It may for example be that assay sensitivity or specificity, respectively, has to be set to certain limits, e.g. 80%, 90%, 95% or 98%, respectively. These requirements may also be defined in terms of positive or negative predictive values. Nonetheless, based on the teaching given in the present invention it will always be possible for a skilled artisan to arrive at the reference level meeting those requirements. In one embodiment the reference level is determined in reference samples from healthy individuals. The reference level in one embodiment has been predetermined in reference samples from the disease entity to which the patient belongs. In certain embodiments the reference level can e.g. be set to any percentage between 25% and 75% of the overall distribution of the values in a disease entity investigated. In other embodiments the reference level can e.g. be set to the median, tertiles or quartiles as determined from the overall distribution of the values in reference samples from a disease entity investigated. In one embodiment the reference level is set to the median value as determined from the overall distribution of the values in a disease entity investigated. The reference level may vary depending on various physiological parameters such as age, gender or subpopulation, as well as on the means used for the determination of the biomarkers referred to herein. In one embodiment, the reference sample is from essentially the same type of cells, tissue, organ or body fluid source as the sample from the individual or patient subj ected to the method of the invention, e.g. if according to the invention blood is used as a sample to determine the level of biomarkers in the individual, the reference level is also determined in blood or a part thereof.

In certain embodiments, the term "larger than the reference level" or "above the reference level" refers to a level of the biomarker in the sample from the individual or patient above the reference level or to an overall increase of 5%, 10%, 20%, 25%, 30%, 40%, 50%, 60%, 70%, 80%, 85%, 90%, 95%, 100% or greater, determined by the methods described herein, as compared to the reference level. In certain embodiments, the term increase refers to the increase in biomarker level in the sample from the individual or patient wherein, the increase is at least about 1.5-, 1.75-, 2-, 3-, 4-, 5-, 6-, 7-, 8-, 9-, 10-, 15-, 20-, 25-, 30-, 40-, 50-, 60-, 70-, 75-, 80-, 90-, or 100- fold higher as compared to the reference level, e.g. predetermined from a reference sample.

In certain embodiments, the term "lower than the reference level" or "below" herein refers to a level of the biomarker in the sample from the individual or patient below the reference level or to an overall reduction of 5%, 10%, 20%, 25%, 30%, 40%, 50%, 60%, 70%, 80%, 85%, 90%, 95%, 96%, 97%, 98%, 99% or greater, determined by the methods described herein, as compared to the reference level. In certain embodiments, the term decrease in biomarker level in the sample from the individual or patient wherein the decreased level is at most about 0.9-, 0.8-, 0.7-, 0.6-, 0.5-, 0.4-, 0.3-, 0.2-, 0.1-, 0.05-, or 0.01- fold of the reference level, e.g. predetermined from a reference sample, or lower.

Preferably, the following applies as diagnostic algorithm:
(a1) a level of C-terminal proSP-B or proSP-B of larger than the reference level for said C-terminal proSP-B or proSP-B, a level of a BNP-type peptide of lower than the reference level of said BNP-type peptide, and a level of KL-6 of larger than the reference level for said KL-6 is indicative for a pulmonary disease with pulmonary edema,
(a2) a level of C-terminal proSP-B or proSP-B of larger than the reference level for said C-terminal proSP-B or proSP-B, a level of a BNP-type peptide of lower than the reference level of said BNP-type peptide, and a level of KL-6 of lower than the reference level for said KL-6 is indicative for a pulmonary disease without pulmonary edema,
(b1) a level of C-terminal proSP-B or proSP-B of lower than the reference level for said C-terminal proSP-B or proSP-B, a level of a BNP-type peptide of larger than the reference level of said BNP-type peptide, and a level of KL-6 of larger than the reference level for said KL-6 is indicative for a cardiovascular complication with pulmonary edema and/or congestion,
(b2) a level of C-terminal proSP-B or proSP-B of lower than the reference level for said C-terminal proSP-B or proSP-B, a level of a BNP-type peptide of larger than the reference level of said BNP-type peptide, and a level of KL-6 of lower than the reference level for said KL-6 is indicative for a cardiovascular complication without pulmonary edema and/or (in particular "and") congestion,
(c1) a level of C-terminal proSP-B or proSP-B of larger than the reference level for said C-terminal proSP-B or proSP-B, a level of a BNP-type peptide of larger than the reference level of said BNP-type peptide, and a level of KL-6 of larger than the reference level for said KL-6 is indicative for a cardiovascular complication accompanied by a pulmonary disease with pulmonary edema and/or congestion,
(c2) a level of C-terminal proSP-B or proSP-B of larger than the reference level for said C-terminal proSP-B or proSP-B, a level of a BNP-type peptide of larger than the reference level of said BNP-type peptide, and a level of KL-6 of lower than the reference level for said KL-6 is indicative for a cardiovascular complication accompanied by a pulmonary disease without pulmonary edema and/or (in particular "and") congestion, and/or
(d) a level of C-terminal proSP-B or proSP-B of lower than the reference level for said C-terminal proSP-B or proSP-B and a level of a BNP-type peptide of lower than the reference level of said BNP-type peptide is indicative for acute dyspnea without cardiovascular or pulmonary causes.

As set forth above, the reference level may be a predetermined level. Such reference levels can be determined by the skilled person without further ado based on the desired specificity and/or the desired sensitivity. Preferably, the reference level for C-terminal proSP-B or proSP-B, the reference level for the BNP-type peptide and the reference level for KL-6 shall allow for the differentiation according to the method of the present invention.

A preferred reference level for C-terminal proSP-B for the differentiation may be within the range of 250 to 500 ng/ml, or, in particular, 250 to 350 ng/ml. A particular preferred reference level is 350 ng/ml, or, in particular, 250 ng/ml.

A preferred reference level for proSP-B for the differentiation is within the range of 80 to 150 ng/ml, or, in particular, 100 to 130 ng/ml. A particular preferred reference level is 100 ng/ml or, in particular, 130 ng/ml.

A preferred reference level for a BNP-type peptide, in particular for NT-proBNP for the differentiation is within the range of 125 to 500 pg/ml, or, in particular, 200 to 300 pg/ml. A particular preferred reference level is 200 pg/ml or, in particular, 300 pg/ml.

A preferred reference level for KL-6 for the differentiation is within the range of 250 to 500 U/ml, or, in particular, 300 to 400 U/ml. A particular preferred reference level is 400 U/ml, or, in particular, 300 U/ml (units per ml).

Advantageously, it has been shown in the context of the studies underlying the present invention that the combined determination of i) C-terminal proSP-P or proSP-P, ii) a BNP-type peptide and iii) KL-6 allows for a reliable differentiation of the causes of acute shortness of breath in a patient who suffers from acute shortness of breath. In particular, it can be differentiated between (a) a pulmonary disease with (a1) pulmonary edema or without (a2) pulmonary edema, (b) a cardiovascular complication with (b1) pulmonary edema and/or congestion, or without (b2) pulmonary edema and/or congestion, (c) a cardiovascular complication accompanied by a pulmonary disease with (c1) pulmonary edema and/or congestion, or without (c2) pulmonary edema and/or congestion, and (d) acute dyspnea without cardiovascular or pulmonary causes. The present invention is a particular advantageous in severely ill patients in which conventional diagnostic measures cannot be carried out. Further, the additional differentiation between the presence of pulmonary edema (and congestion) and the absence of pulmonary edema is advantageous since it allows for better selection of suitable treatment regimes for treating the underlying disease in a patient who suffers from acute shortness of breath.

In an embodiment of the present invention, the aforementioned method further comprises the step of recommending a suitable treatment. Preferably, the recommendation is based on the results of the differentiation.

The phrase "recommending a treatment" as used herein refers to using the information or data generated relating to the levels of the biomarkers as measured in accordance with the method of the present invention in a sample of the patient for proposing or selecting a therapy for the patient. The information or data used or generated may be in any form, written, oral or electronic. In some embodiments, using the information or data generated includes communicating, presenting, reporting, storing, sending, transferring, supplying, transmitting, dispensing, or combinations thereof. In some embodiments, communicating, presenting, reporting, storing, sending, transferring, supplying, transmitting, dispensing, or combinations thereof are performed by a computing device, analyzer unit or combination thereof.

If the method of the present invention indicates that the patient suffers from pulmonary edema, then the patient preferably shall receive mechanical ventilatory support. If the pulmonary edema is non-cardiogenic edema, fluid restriction and diuretics might be applied.

If the method of the present invention indicates that the patient suffers from a cardiovascular complication, in particular heart failure, then the patient is preferably treated with Angiotensin-converting enzyme (ACE) inhibitors and beta-blockers. Mineralocorticoid receptor antagonist might recommended as well. The ACE inhibitors have a modest effect on remodeling of the particular left ventricular heart function and the beta-blockers are supposed to improve the cardiac ejection fraction. In patients with signs and symptoms of congestion and edema diuretics will be added to those medications in order to relieve dyspnea and edema. (ESC Guideline 2012, European Journal of Heart Failure (2012) 14, 803-869 which is hereby incorporated by reference in its entirety).

Also envisaged by the present invention is a method for treating a patient suffering from acute shortness of breath, the method comprising steps i) to iv) of the aforementioned method and treating a patient as indicated above.

The definitions given herein above, preferably, apply mutatis mutandis to the following.

### Method for monitoring a patient suffering from acute shortness of breath.

The present invention further relates to method for monitoring a patient suffering from acute shortness of breath, said method comprising
(a) measuring the level of C-terminal proSP-B or proSP-B in a first and second sample of said patient,
(b) measuring the level of a BNP-type peptide in a first and second sample of said subject,
(c) measuring the level of a KL-6 in a first and second sample of said subject, and
(d) comparing the level of the biomarker in the second sample as measured in the steps i), ii) and iii), respectively, to the level of said biomarker in said first sample as measured in steps i), ii), and iii), respectively.

In an embodiment, the level of the biomarkers as referred to herein is measured by contacting the sample with an agent that specifically binds to the respective marker, thereby form-ing a complex between the agent and said marker, detecting the amount of complex formed, and thereby measuring the level of said biomarker.

The term "monitoring" as used herein, preferably, refers to keeping track of a patient who suffers from acute shortness of breath. Preferably, monitoring said patient, it can be determined whether the condition of a patient improves or deteriorates. In particular, it can be monitored whether a a) cardiovascular complication, b) a pulmonary disease and/or c) pulmonary edema and/or congestion deteriorates or improves.

As will be understood by those skilled in the art, such an assessment is usually not intended to be correct for 100% of the patients to be monitored. The term, however, requires that the assessment is correct for a statistically significant portion of the patients (e.g. a cohort in a cohort study). Whether a portion is statistically significant can be determined without further ado by the person skilled in the art using various well known statistic evaluation tools, e.g., determination of confidence intervals, p-value determination, Student's t-test, Mann- Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1 , 0.05, 0.01 , 0.005, or 0.0001.

The "first sample" can be obtained from the patient at any time after the onset of acute shortness of breath. In an embodiment, it is envisaged that the sample is obtained within the first 24 hours after the onset of acute shortness of breath. In another embodiment, it is envisaged that the sample is obtained on the second day or the third day after the onset. However, it is also contemplated that the sample is obtained later such as on the 5th day or the 7th day after the onset.

Preferably, the "second sample" has been obtained after the first sample. The sample is, preferably, understood as a sample which is obtained in order to reflect a change of the level of the respective marker as compared to the level of the respective marker in the first sample. Preferably, the second sample is not obtained too early after the first sample (in order to observe a sufficiently significant change to allow for monitoring). Preferably, the sample has been obtained at least one day, more preferably, at least two days, and, most preferably, at least three days after the first sample.

Also preferably, the second sample has been obtained not more than 10 days of after the first sample.

Preferably, the patient suffers from acute shortness of breath at the time points at which both samples, i.e. the first and the second sample, are obtained.

Preferably, an increase of the level of C-terminal proSP-B or proSP-B in the second sample as compared to the first sample as measured in step i) is indicative for a deterioration of pulmonary disease, whereas a decrease of C-terminal proSP-B or proSP-B in the second sample as compared to the first sample is indicative for an improvement of pulmonary disease.

Also preferably, an increase of the level of the BNP-type peptide in the second sample as compared to the first sample as measured in step ii) is indicative for a deterioration of a cardiovascular complication, whereas a decrease of the level of said BNP-type peptide in the second sample as compared to the first sample is indicative for an improvement of a cardiovascular complication.

Also preferably, an increase of the level of KL-6 in the second sample as compared to the first sample as measured in step iii) is indicative for a deterioration of pulmonary edema and/or congestion, whereas a decrease of the level of said BNP-type peptide in the second sample as compared to the first sample is indicative for an improvement of pulmonary edema and/or congestion.

Thus, an increase, and more preferably a statistically significant increase of the level of the biomarker in said second sample compared to the level in said first sample indicates deterioration of the condition(s) (pulmonary disease, if the marker is C-terminal proSP-B or proSP-B; cardiovascular complication if the marker is a BNP-type peptide, and pulmonary edema and/or congestion, if the marker is KL-6) and, thus, that the condition(s) has (have) deteriorated during the period between obtaining the first and the second sample. Thus, there was a progression of the condition.

Preferably, a decrease of the level of the biomarker in said second sample as compared to said first sample indicates improvement of said condition(s) and, thus, that the condition(s) has (have) improved during the period between obtaining the first and the second sample. Thus, there was regression of said condition.

Particularly, a significant increase (or decrease) is an increase (or decrease) of a size which is considered to be significant for, particularly statistically significant. The terms "significant" and "statistically significant" are known to the person skilled in the art. Whether a change or an increase (or decrease) is statistically significant can be determined without further ado by the person skilled in the art using various well known statistic evaluation tools including those referred to herein.

Moreover, an unchanged level, preferably an essentially unchanged level, in said second sample as compared to said first sample, preferably, indicates no change in status of said condition(s). Thus, the condition(s) of the patient was (were) stable.

Preferred significant increases of the levels of the biomarker which have been found in the course of the invention which indicate that conditions has deteriorated are given below. According to the invention, an increase of the level of respective in the second sample compared to the level in the first sample, preferably, of at least 10% more preferably of at least 30 % and most preferably, of at least 50 % considered to be significant and, thus, to be indicative for a deterioration of the condition.

Preferably, a decrease of the level of the biomarker in the second sample compared to the level in the first sample, preferably, of at least 10% more preferably of at least 20% and, most preferably, of at least 30% is considered to be significant and, thus, to be indicative for an improvement of the condition.

Preferred (significant) increases being indicative for a deterioration of a condition as referred to herein are (in increasing order of preference):
- at least 150 ng/ml or at least 300 ng/ml if the biomarker is C-terminal proSP-B
- at least 40 ng/ml or at least 100 ng/ml if the biomarker is proSP-B
- at least 150 pg/ml or at least 300 pg/ml if the biomarker is NT-proBNP
- at least 100 U/ml or at least 250 U/ml if the biomarker is KL-6

Preferred (significant) decreases being indicative for an improvement of a condition as referred to herein are (in increasing order of preference):
- at least 100 ng/ml or at least 200 ng/ml if the biomarker is C-terminal proSP-B
- at least 40 ng/ml or at least 100 ng/ml if the biomarker is proSP-B
- at least 150 pg/ml or at least 300 pg/ml if the biomarker is NT-proBNP
- at least 100 U/ml or at least 250 U/ml if the biomarker is KL-6

The definitions and explanations given herein above, also apply to the following embodiments of the present invention.

Method for diagnosing whether a patient who suffers from a acute shortness of breadth has pulmonary edema and/or congestion

The present invention further contemplates a method for diagnosing whether a patient who suffers from a acute shortness of breath has pulmonary edema and/or congestion, said method comprising the steps:
(a) measuring the level of a KL-6 in a sample of said patient,
(b) comparing the level of KL-6 to a reference level, and optionally,
(c) providing a diagnosis whether the patent has pulmonary edema and/or congestion.

In an embodiment of the aforementioned method of the present invention, a level of KL-6 above the reference level is indicative for a patient who has pulmonary edema and/or congestion, whereas a level of KL-6 below the reference level is indicative for a patient who does not have pulmonary edema and/or (in particular "and") congestion.

In an embodiment, the level of the biomarker KL-6 is measured by contacting the sample with an agent that specifically binds to said biomarker, thereby forming a complex between the agent and said biomarker, detecting the amount of complex formed, and thereby measuring the level of said biomarker.

The terms "pulmonary edema" and "congestion" are defined elsewhere herein. Preferably, the term includes non-cardiogenic and cardiogenic pulmonary edema.

The term "reference level" has been defined above. In an embodiment, the reference level may be a predetermined level, in particular a level which allows for differentiating between a patient who has pulmonary edema and/or congestion and/or a patient who does not have pulmonary edema and/or (in particular "and") congestion. Such reference levels can be determined by the skilled person without further ado based on the desired specificity and/or the desired sensitivity.

A preferred reference level for KL-6 for the diagnosis is within the range of 250 to 500 U/ml, or, in particular, 300 to 400 U/ml. A particular preferred reference level is 400 U/ml, or, in particular, 300 U/ml (units per ml).

The present invention also relates to the use of
x) C-terminal proSP-B or proSP-B,
y) a BNP-type peptide and
z) KL-6
and/or of
x1) a binding agent which specifically binds to C-terminal proSP-B or proSP-B,
y1) a binding agent which specifically binds to a BNP-type peptide, and
z1) a binding agent which specifically binds to KL-6
in a sample of a patient who suffers from acute shortness of breath for differentiating between
(a) a pulmonary disease with (a1) pulmonary edema or without (a2) pulmonary edema,
(b) a cardiovascular complication with (b1) pulmonary edema and/or congestion, or without (b2) pulmonary edema and/or (in particular "and") congestion,
(c) a cardiovascular complication accompanied by a pulmonary disease with (c1) pulmonary edema and/or congestion, or without (c2) pulmonary edema and/or (in particular "and") congestion, or
(d) acute dyspnea without cardiovascular or pulmonary causes.

Also envisaged is the use of
x) C-terminal proSP-B or proSP-B,
y) a BNP-type peptide and
z) KL-6
and/or of
x1) a binding agent which specifically binds to C-terminal proSP-B or proSP-B,
y1) a binding agent which specifically binds to a BNP-type peptide, and
z1) a binding agent which specifically binds to KL-6
for the manufacture of a diagnostic of pharmaceutical preparation for differentiating in a patient who suffers from acute shortness of breath between
(a) a pulmonary disease with (a1) pulmonary edema or without (a2) pulmonary edema,
(b) a cardiovascular complication with (b1) pulmonary edema and/or congestion, or without (b2) pulmonary edema and/or (in particular "and") congestion,
(c) a cardiovascular complication accompanied by a pulmonary disease with (c1) pulmonary edema and/or congestion, or without (c2) pulmonary edema and/or (in particular "and") congestion, or
(d) acute dyspnea without cardiovascular or pulmonary causes.

The present invention also relates to the use of
x) C-terminal proSP-B or proSP-B,
y) a BNP-type peptide and
z) KL-6
and/or of
x1) a binding agent which specifically binds to C-terminal proSP-B or proSP-B,
y1) a binding agent which specifically binds to a BNP-type peptide, and
z1) a binding agent which specifically binds to KL-6
in a first and second sample from a patient who suffers from acute shortness of breath for monitoring said patient.

Further envisaged is the use of
x) C-terminal proSP-B or proSP-B,
y) a BNP-type peptide and
z) KL-6
and/or of
x1) a binding agent which specifically binds to C-terminal proSP-B or proSP-B,
y1) a binding agent which specifically binds to a BNP-type peptide, and
z1) a binding agent which specifically binds to KL-6
for the manufacture of a diagnostic of pharmaceutical preparation for monitoring a patient who suffers from acute shortness of breath.

As set forth above, the binding agent may be an antibody.

Preferably, the aforementioned uses are *in vitro* uses.

According to a preferred embodiment of the present invention, a device adapted for carrying out a method of the invention is provided comprising
i) an analyzer unit comprising a binding agent which specifically binds to C-terminal proSP-B or proSP-B, a binding agent which specifically binds to a BNP-type peptide, and a binding agent which specifically binds to KL-6, said unit being adapted for measuring the levels of the biomarkers in a sample of a patient having acute shortness of breath, and
ii) an analyzer unit for comparing the determined levels with reference levels,
   whereby it is differentiated between
   (a) a pulmonary disease with (a1) pulmonary edema or without (a2) pulmonary edema,
   (b) a cardiovascular complication with (b1) pulmonary edema and/or congestion, or without (b2) pulmonary edema and/or congestion,
   (c) a cardiovascular complication accompanied by a pulmonary disease with (c1) pulmonary edema and/or congestion, or without (c2) pulmonary edema and/or congestion, or
   (d) acute dyspnea without cardiovascular or pulmonary causes,
   said unit comprising a database with reference levels for the respective biomarkers and a computer-implemented algorithm for carrying out the comparison.

Preferred reference levels and algorithms are disclosed elsewhere herein.

A preferred embodiment of the instant disclosure includes a system for identifying a patient as likely to respond to a therapy comprising a statin. Examples of systems include clinical chemistry analyzers, coagulation chemistry analyzers, immunochemistry analyzers, urine analyzers, nucleic acid analyzers, used to detect the result of chemical or biological reactions or to monitor the progress of chemical or biological reactions. More specifically, exemplary systems of the instant disclosure may include Roche Elecsys™ Systems and Cobas^{®} e Immunoassay Analyzers, Abbott Architect™ and Axsym™ Analyzers, Siemens Centaur™ and Immulite™ Analyzers, and Beckman Coulter UniCel™ and Acess™ Analyzers, or the like.

Embodiments of the system may include one or more analyzer units utilized for practicing the subject disclosure. The analyzer units of the system disclosed herein are in operable communication with the computing device disclosed herein through any of a wired connection, Bluetooth, LANS, or wireless signal, as are known. Additionally, according to the instant disclosure, an analyzer unit may comprise a stand-alone apparatus, or module within a larger instrument, which performs one or both of the detection, e.g. qualitative and/or quantitative evaluation of samples for diagnostic purpose. For example, an analyzer unit may perform or assist with the pipetting, dosing, mixing of samples and/or reagents. An analyzer unit may comprise a reagent holding unit for holding reagents to perform the assays. Reagents may be arranged for example in the form of containers or cassettes containing individual reagents or group of reagents, placed in appropriate receptacles or positions within a storage compartment or conveyor. Detection reagents may also be in immobilized form on a solid support which are contacted with the sample. Further, an analyzer unit may include a process and/or detection component which is optimizable for specific analysis.

According to some embodiments, an analyzer unit may be configured for optical detection of an analyte, for example a marker, with a sample. An exemplary analyzer unit configured for optical detection comprises a device configured for converting electro-magnetic energy into an electrical signal, which includes both single and multi-element or array optical detectors. According to the present disclosure, an optical detector is capable of monitoring an optical electro-magnetic signal and providing an electrical outlet signal or response signal relative to a baseline signal indicative of the presence and/or concentration of an analyte in a sample being located in an optical path. Such devices may also include, for example, photodiodes, including avalanche photodiodes, phototransistors, photoconductive detectors, linear sensor arrays, CCD detectors, CMOS detectors, including CMOS array detectors, photomultipliers, and photomultiplier arrays. According to certain embodiments, an optical detector, such as a photodiode or photomultiplier, may contain additional signal conditioning or processing electronics. For example, an optical detector may include at least one pre-amplifier, electronic filter, or integrated circuit. Suitable pre-preamplifiers include, for example, integrating, transimpedance, and current gain (current mirror) preamplifiers.

Additionally, one or more analyzer unit according to the instant disclosure may comprise a light source for emitting light. For example, a light source of an analyzer unit may consist of at least one light emitting element (such as a light emitting diode, an electric powered radiation source such as an incandescent lamp, an electroluminescent lamp, a gas discharge lamp, a high-intensity discharge lamp, a laser) for measuring analyte concentrations with a sample being tested or for enabling an energy transfer (for example, through florescent resonance energy transfer or catalyzing an enzyme).

Further, an analyzer unit of the system may include one or more incubation units (for example, for maintaining a sample or a reagent at a specified temperature or temperature range). In some embodiments, an analyzer unit may include a thermocycler, include a real-time thermocycler, for subjecting a sample to repeated temperature cycles and monitoring a change in the level of an amplification product with the sample.

Additionally, an analyzer unit of the system disclosed herein may comprise, or be operationally connected to, a reaction vessel or cuvette feeding unit. Exemplary feeding units include liquid processing units, such as a pipetting unit, to deliver samples and/or reagents to the reaction vessels. The pipetting unit may comprise a reusable washable needle, e.g. a steel needle, or disposable pipette tips. The analyzer unit may further comprise one or more mixing units, for example a shaker to shake a cuvette comprising a liquid, or a mixing paddle to mix liquids in a cuvette, or reagent container.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art. The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities. According to some embodiments of the instant disclosure, an algorithm for carrying out a comparison between a determined level of one or more markers disclosed herein, and a suitable reference (in particular, the level in the first sample), is embodied and performed by executing the instructions. The results may be given as output of parametric diagnostic raw data or as absolute or relative levels. According to various embodiments of the system disclosed herein, a "diagnosis" may be provided by the computing device of a system disclosed herein based on said comparison of the calculated "level", to a reference or a threshold, in particular a predetermined level as set forth elsewhere herein. For example, a computing device of a system may provide an indicator, in the form of a word, symbol, or numerical value which is indicative of a particular diagnosis.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Finally, the present invention relates to a kit comprising a) a binding agent which specifically binds to C-terminal proSP-B or proSP-B, b) a binding agent which specifically binds to a BNP-type peptide, and c) a binding agent which specifically binds to KL-6. Also envisaged is the use of said kit for differentiating between the various causes of acute shortness of breath (i.e. between (a1), (a2), (b1), (b2), (c1), (c2) and (d) as set forth herein elsewhere), in particular in a sample of a patient who suffers from acute shortness of breath. Preferably, said use is an *in vitro* use.

In an embodiment, the binding agent is an antibody.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a pre-defined level for the biomarker as referred to herein representing a reference level.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilized in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example 1: Assays

### Measurements were done in serum samples

The biomarkers proSP-B and C-terminal proSP-B were measured as disclosed in Example 1 and 2 of WO 2012/130731.

NT-proBNP was determined in samples using Roche's electrochemiluminescence ELISA sandwich test Elecsys proBNP II STAT (Short Turn Around Time) assay. The test employs two monoclonal antibodies which recognize epitopes located in the N-terminal part (1-76) ofproBNP (1-108).

KL-6 was determined by using the Nanopia KL-6 assay which is based on a latex immuno-turbidimetric assay. The kit is manufactured by Sanko Junyaku Co., Ltd.

### Example 2: Case studies

### Case ID 24

| **61 years old male patient** | | | | |
|---|---|---|---|---|
| | **Timepoint 1** | **Timepoint 2** | **Time point 3** | |
| | 0 | 48 | 216 | hours |
| | 0 | 2 | 9 | days |
| | | | | |
| CproSP-B* | 275.3 | 329.4 | 150.9 | ng/m L |
| proSP-B | 49.75 | 14.34 | 7.4 | ng/m L |
| KL-6 | 264 | 385 | 522 | U/mL |
| proBNP | 239.24 | 131.79 | 902.81 | pg/m L |
| | | | | |

| **Infection/Inflammation** | | | | |
|---|---|---|---|---|
| CRP (C-reactive protein) | 67.58 | 50.92 | 18.57 | mg/L |
| Leukocytes | nd | | | per nL |
| | | | | |

| **Renal function** | | | | |
|---|---|---|---|---|
| Cystatin C | 5.71 | 4.35 | 5.88 | mg/L |
| Creatinine | 475 | 540 | 408 | µmol/ L |
| | | | | |

| **Blood gase analysis** | | | | |
|---|---|---|---|---|
| PaO2/FiO2 | nd | | | mmH g |
| Apache 2 | nd | | | score points |
| | | | | |
| CproSP-B = C-terminal proSP-B | | | | |

### Description:

61 years old male patient with moderate alveolar injury as judged by the proSP-B levels (275. 3 ng/mL at admission and 329 ng/mL at timepoint 2). Patient had likely acutely suffered from infection which resolved (CRP values declining). At timepoint 3 the C-proSP-B levels are back to normal indicating a healing process of the alveolar injury. The patient, however suffers likely from chronic kidney disease with constantly high Cystatin C and Creatinine at all 3 timepoints. At timepoint , after 9 days of ARDS diagnosis and treatment initiation of ARDS, the patient develops acute heart insufficiency indicated by proBNP levesl jumping from 132 pg/mL at timepoint 2 (normal range) to 903 pg/mL at timepoint 3. At the same time KL-6 raises from 385 to 522 U/mL indicating that the interstitium is affected by blood congestion back into the lung from the heart and possibly edema has developed. By judging the biomarker combination or C-proSP-B, KL-6 and proBNP, it can be derived that the alveolar injury has healed (C-proSP-B back to normal), but that the patient has developed a lung interstitial infiltration due to acute heart insufficiency.

### Case ID 21

| **69 years old female patient** | | | | |
|---|---|---|---|---|
| | **Timepoint 1** | **Timepoint 2** | **Timepoint 3** | |
| | 0 | 96 | 168 | hours |
| | 0 | 4 | 7 | days |
| | | | | |
| CproSP-B | 400.4 | 605.4 | 340.3 | ng/mL |
| proSP-B | 21.65 | 47.8 | 22.65 | ng/mL |
| KL-6 | 337 | 910 | 1329 | U/mL |
| proBNP | 2188.24 | 4137 | 4137 | pg/mL |
| | | | | |
| | | | | |

| **Infection** | | | | |
|---|---|---|---|---|
| CRP | 349.16 | 229.35 | 143.68 | mg/L |
| Leukocytes | nd | | | per nL |
| | | | | |
| | | | | |

| **Renal function** | | | | |
|---|---|---|---|---|
| Cystatin C | 5.31 | 5.87 | 6.81 | mg/L |
| Creatinine | 204 | 349 | 441 | µmol/L |
| | | | | |
| | | | | |

| **Blood gase analysis** | | | | |
|---|---|---|---|---|
| PaO2/FiO2 | 66 | | | mmHg |
| Apache 2 | 20 | | | score points |

### Description:

Patient with severe ARDS at timepoint 0 with PaO2/FiO2 of 66 mm Hg only. The C-proSP-B levels are reflecting the physiological course of ARDS and injury of the alveolar surfactant layer. While from timepoint 1 to 2 the concentration is raising, it is decreasing towards timepoint 3 indicating a healing process of the alveolar layer. Initial course of ARDS could be an infection, which improves (CRP declining). The patient, however, suffers from heart insufficiency (proBNP levels of 2188.24 pg/mL) at timepoint 1 and develops acute heart failure/acute cardio-renal failure with proBNP values jumping to 4137 pg/mL and Cystatin C > 6 mg/mL and Creatinine > 440 u mol/L at timepoints 2and 3. KL-6 values are increasing to 1329 U/mL at timepoint 3 as a consequence of the raised backwards pressure leading to congestion and pulmonary edema. An x-ray taken at timepoint 3 would possibly have confirmed this assumption, but is not always possible to be taken in those severely ill patients. While the differentiation of the cause of a pulmonary edema may not always be possible from a chest X-ray (i. e. the differentiation of a cardiogenic versus non-cardiogenic pulmonary edema in a patient suffering from cardiac and pulmonary disease) and typical lung function testing cannot be performed in such critical ill patients, the combined assessment of the 3 biomarkers (C-proSP-B, KL-6, proBNP) may allow for this differentiation. Interestingly, the proSP-B levels in this patient are in the normal range and show very little variation.

### Case ID 31

| **74 year-old female patient, 165 cm, 56 kg, smoker, 35 pack years** | | | | |
|---|---|---|---|---|
| **COPD** | | | | |
| | **Day 1** | **Day 3** | **Day 7** | |
| CproSP-B | 2733 | 3423 | 3623 | ng/mL |
| proSP-B | 427 | 428 | 456 | ng/mL |
| KL-6 | 940 | 1182 | 1079 | U/mL |
| proBNP | 3656.37 | 6640.57 | 6212.82 | pg/mL |
| | | | | |

| **Infection/Inflammation** | | | | |
|---|---|---|---|---|
| CRP | | 72 | 31.9 | mg/L |
| Leukocytes | | 14.88 | 10.59 | per nL |
| Temperature | 37.6 | 37.2 | 36.2 | °C |
| | | | | |

| **Coagulation/Hematology** | | | | |
|---|---|---|---|---|
| INR | | 0.95 | 0.94 | |
| PTT | | 24.3 | 24 | s |
| PLT | | 291 | 276 | per nL |
| Erys | | 3.85 | 3.86 | per nL |
| Hb | | 11.9 | 12.1 | g/dL |

| **Renal function** | | | | |
|---|---|---|---|---|
| Cystatin C | 2.018 | 1.784 | 1.702 | mg/L |
| Creatinine | | 1.38 | 1.05 | µmol/L |
| | | | | mL/min/1.73 |
| **Glomerular Filtration Rate (GFR)** | | 40 | 54 | m2 |
| | | | | |

| **Blood gase analysis, Electrolytes** | | | | |
|---|---|---|---|---|
| pH | 7.477 | | 7.462 | |
| pO2 | 49.4 | | 66.1 | mmHg |
| pCO2 | 34.4 | | 37.3 | mm Hg |
| SaO2 | 87.2 | | 93.7 | % |
| BE | 2.4 | | 3 | meq/L |
| Na | | 126 | 125 | µmol/L |
| Cl | | 89 | 88 | µmol/L |
| K | | 3.13 | 3.58 | µmol/L |
| | | | | |
| | | | | |

| **Lung function testing** | | | | |
|---|---|---|---|---|
| FEV1 | 1.15 | | 0.93 | L |
| FEV1 % | 55.4 | | 45.1 | % |
| FVC | 2.12 | | 1.8 | L |
| TLC | 5.78 | | 4.75 | L |
| RV | 3.49 | | 2.95 | L |
| DLCO | 0.53 | | | |
| Bode Index | | 7 | 6 | |
| | | | | |

| **Cardiac function** | | | | |
|---|---|---|---|---|
| | | | | |
| ECG | | | | |
| PQ | | 180 | | ms |
| QRS | | 90 | | ms |
| QT | | 360 | | ms |
| Heart rate | | 92 | | beats per minute |

### Description:

The 74 years-old female patient suffers from a known chronic obstructive lung disease (COPD). The patient is smoker with a consumption of 35 packyears. The patient presents with a medium dyspnea (FEV1 55%) and the x-ray done on admission show signs of pulmonary congestion, but no signs of edema or infiltration yet.

The proSP-B levels are highly elevated and increase further during hospital stay. Likewise raise KL-6 and proBNP. The later indicates that the patient preexisting heart insufficiency has acutely worsened. In a chest x-ray done at timepoint 2 or 3 a pulmonary edema would have likely been seen. However, to keep the x-ray exposure minimal for a patient, chest x-rays are frequently done on admission, but not necessarily thereafter. The blood biomarker combination proSP-B, KL-6, and proBNP could therefore help monitoring patients and to early on recognizing an aggravation of the cardiopulmonary status of a patient and identifing the cause of the aggravation which will help to adjust therapy timely. In this case a patient with a chronic lung disease has developed acute heart failure on top of an existing heart insufficiency with pulmonary congestion and likely edema as judged by the raising KL-6 levels in parallel to NT-proBNP.

### Case ID 209

### 54 years old male patient, 97 kg, smoker until 1984

Patient presents with mild dyspnea according to FEV 1 (79%), pO2 reduced (66 mmHg), Sa02a mildly reduced (95%)

| **TestName** | **studyUnit** | **ResultStudyUnit** |
|---|---|---|
| C-proSP-B | ng/mL | 197.8 |
| KL-6_Seki | U/mL | 790 |
| proSP-B | ng/mL | 110.4 |
| NT-proBNP | pg/mL | 16675 |

### Description:

The 54 years old patient is a multimorbid patient with a massively reduced cardiac function, mainly of the right ventricle. The patient is known to suffer from mitral valve and aortic valve insufficiency. Due to this heart valve insufficiency of the left heart the patient very likely suffers from congestion and raised blood pressure in the pulmonal veins which possibly lead to the right heart insufficiency. Coronary artery disease is known, the patient has a heart pacemaker. The diabetic and insulin-dependent patient suffers from end-stage chronic kidney disease (dialysis) and is newly diagnosed of pulmonary hypertension.

The massive cardiac hypertrophy and heart insufficiency is reflected in the very high NT-proBNP value (16675). The heart insufficiency is affecting the interstitium of the alveolar-capillary membrane) and the increased KL-6 levels of 790 U/mL are very likely a consequence of the severely reduced heart function leading to pulmonary congestion and edema while the surfactant layer of the alveolar membrane is still intact (C-proSP-B 198 ng/mL).

### Case ID 230

**41 years old female patient, 71 kg , pulmonary function according to FEV1% and SaO2 within normal range, but pO2 slightly reduced**

| **TestName** | **StudyUnit** | **ResultStudyUnit** |
|---|---|---|
| C-proSP-B | ng/mL | 198.7 |
| KL-6_Seki | U/mL | 364 |
| proSP-B | ng/mL | 143.5 |
| NT-proBNP | pg/mL | 56 |

### Description:

Patient has a mildly hypertrophic right ventricle with otherwise normal cardiac function consistent with NTproBNP in normal range. There are no signs of congestion or pulmonary edema, neither cardiogenic nor non-cardiogenic, but there is steeply raising pressures of the pulmonary artery reported in the patient in situations of hypoxia. Patient is diagnosed of a restrictive lung disease of unkown origin, but without manifest pulmonary hypertension and otherwise normal lung function. The very mildly elevated KL-6 value of 364 U/ mL could be a sign of the occasionally occurring raised pulmonary artery pressures, however, the patient has no pulmonary cardiogenic or noncardiogenic edema and an intact surfactant layer which is consistent with the normal values of the 3 markers.

### Case ID 33

| 77 years-old male patient, 172 cm, 74 kg (BMI), ex smoker, 15 pack years | | | | |
|---|---|---|---|---|
| Heart failure | | | | |
| | **Day 1** | **Day 3** | **Day 7** | |
| CproSP-B | 563 | 598 | 357 | ng/mL |
| proSP-B | 348 | 424 | 153 | ng/mL |
| KL-6 | 348.3 | 345 | 362 | U/mL |
| proBNP | 3087 | 7262 | 9317 | pg/mL |
| | | | | |

| **Infection** | | | | |
|---|---|---|---|---|
| CRP | 11.8 | 137 | 139 | mg/L |
| Leukos | 8.54 | 11.62 | 8.93 | per nL |
| Temp | 36.4 | 36.6 | 36.2 | °C |
| | | | | |

| **Coagulation Hematology** | | | | |
|---|---|---|---|---|
| INR | 2.92 | 2.67 | 3.05 | |
| PTT | | | | s |
| PLT | 138 | 110 | 94 | per nL |
| Erys | 5.05 | 4.9 | 5.11 | per nL |
| Hb | 14.6 | 14 | 14.2 | g/dL |
| | | | | |

| **Renal Function** | | | | |
|---|---|---|---|---|
| Cystatin C | 2.09 | 2.63 | 2.32 | mg/L |
| Creatinine | 2.22 | 2.53 | 2.2 | µmol/L |
| | | | | mL/min/ |
| GFR | 31 | 26 | 31 | 1.73 m2 |
| | | | | |

| **Blood gases, electrolytes** | | | | |
|---|---|---|---|---|
| **pH** | 7.465 | | 7.426 | |
| pO2 | 66.7 | | 49.6 | mmHg |
| pCO2 | 36.7 | | 33.8 | mm Hg |
| SaO2 | 93.9 | | 85.9 | % |
| BE | 2.9 | | -1.3 | meq/L |
| Na | 139 | 136 | 138 | µmol/L |
| Cl | 98 | 97 | 102 | µmol/L |
| K | 3.9 | 4.2 | | 3.9 µmol/L |
| | | | | |
| Albumin | 34.8 | 31.5 | 27.1 | |
| | | | | |

| **Lung function testing** | | | | |
|---|---|---|---|---|
| FEV1 | 2.14 | | 1.8 | L |
| FEV1 % | 80 | | 67 | % |
| FVC | 2.88 | | 2.47 | L |
| TLC | | 5.13 | 5.03 | L |
| RV | | 2.26 | 2.43 | L |
| DLCO | | 4.72 | 3.95 | |
| Bode Index | | 6 | 6 | |
| | | | | |

| **Cardiac function** | | | | |
|---|---|---|---|---|
| **ECG** | | | | |
| PQ | | 0 | | ms |
| QRS | | 160 | | ms |
| QT | | 420 | | ms |
| Rhythmus | SM | | | beats/m |
| HR | | 94 | | |
| | | | | |
| **ECHO** | not available | | | |

### Description:

77 year old male patient with almost normal lung function as judged by FEV 1 %, but signs of pulmonary congestion and cardiomegaly in the x-ray on admission. The patient has likely developed an infection (elevated CRP at day 3 and 7). The C-proSP-B values are elevated at admission, but decrease during stay in hospital. The KL-6 values are mildly elevated and remain almost constant during the same time indicating congestion, but without signs of edema or infiltration in the chest x-ray despite a cardiomegalia. The increased NT-proBNp values are consistent with the cardiomegalia as a sign of heart insufficiency, but with only moderate congestion and no pulmonary edema consistent with the KL-6 value. Possibly the patient as suffered from a respiratory-pulmonary infection which has affected caused alveolar surface as indicted by the increased C-proSP-B levels and caused the dyspnea.

### Case ID 27

| **54 years old male patient** | | | | |
|---|---|---|---|---|
| | **Timepoint 1** | **Timepoint 2** | **Timepoint 3** | |
| | 0 | 48 | 96 | hours |
| | 0 | 2 | 4 | days |
| | | | | |
| CproSP-B | 1031 | 833.5 | 751.5 | ng/mL |
| proSP-B | 390.4 | 374.6 | 368.1 | ng/mL |
| KL-6 | 118 | 156 | 163 | U/mL |
| proBNP | 47.15 | 42.19 | 58.91 | pg/mL |
| | | | | |

| **Inflammation** | | | | |
|---|---|---|---|---|
| CRP | 98.85 | 174.63 | 101.54 | mg/L |
| Leukos | 8.3 | | | per nL |
| | | | | |

| **Renal function** | | | | |
|---|---|---|---|---|
| Cystatin C | 1.12 | 2.81 | 2.66 | mg/L |
| Creatinine | 59 | 202 | 189 | µmol/L |
| | | | | |

| **Blood gase analysis** | | | | |
|---|---|---|---|---|
| PaO2/FiO2 | 123 | | | mmHg |
| Apache 2 | 7 | | | score points |

### Description:

54 years-old male patient with acute respiratory distress syndrome and under ventilatory mechanical support. The ARDS is reflected in the high C-proSP-B values, at timepoint 1 of 1031 ng/mL and declining thereafter. Cause of the respiratory insufficiency is likely an infection (elevated CRP values). The declining C-proSP-B are also indicating treatment effectiveness. Kl-6 and proBNP are within normal range indicating that the respiratory distress of the patient is due to pulmonary disease only and not overlayed by cardiac disease and a pulmonary edema . The biomarker combination of C-proSP-B, KL-6 and proBNP can be used for the differential diagnosis of respiratory distress in severely ill patients and to recognize pulmonary congestion and edema in patients where classical diagnostic methods (chest x-ray, echocardiography) are difficult to perform.

### Case ID 5

| 57 years-old male patient, 161 cm, 65 kg, ex smoker, 8 pack years | | | | |
|---|---|---|---|---|
| ICD J45.0 predominantly Asthma bronchiale | | | | |
| | **Day 1** | **Day 3** | **Day 7** | |
| CproSP-B | 187 | 203.4 | 193.3 | ng/mL |
| proSP-B | 114.9 | 120.2 | 114.5 | ng/mL |
| KL-6 | 156 | 162 | 149 | U/mL |
| proBNP | 82.14 | 77.13 | 238.47 | pg/mL |
| | | | | |

| **Inflammation** | | | | |
|---|---|---|---|---|
| CRP | 16.5 | 2.9 | 25.7 | mg/L |
| Leukos | 6.9 | 6.4 | 9.53 | per nL |
| Temp | 36.4 | 36.2 | 36.4 | °C |
| | | | | |

| **Coagulation/Hematology (Anemia)** | | | | |
|---|---|---|---|---|
| INR | 2.67 | | 1.07 | |
| PTT | 35.8 | | 22.7 | s |
| PLT | 143 | 284 | 172 | per nL |
| Erys | 4.42 | 4.54 | 4.75 | per nL |
| Hb | 13.9 | 11.7 | 14.6 | g/dL |
| | | | | |

| **Renal function** | | | | |
|---|---|---|---|---|
| Cystatin C | 0.866 | 0.866 | 1.01 | mg/L |
| Creatinine | 1.17 | 0.84 | 0.9 | µmol/L |
| GFR | | 80 | 65 | mL/min/1.73 m2 |
| | | | | |

| **Blood gase analysis (Acidosis, alcalosis, gas exchange)** | | | | |
|---|---|---|---|---|
| pH | 7.43 | 6mgt | 7.45 | |
| pO2 | | | 54.3 | mm Hg |
| pCO2 | | | 29 | mmHg |
| SaO2 | 95.4 | | 89.5 | % |
| BE | 2.5 | | -2.4 | meq/L |
| Na | 139 | 137 | 138 | mmol/L |
| Cl | 102 | | | mmol/L |
| K | 4 | 4.43 | 4 | mmol/L |
| Lactate | | | | |
| Albumin | 35.2 | 35.6 | 36.5 | |

| **Lung function testing** | | | | |
|---|---|---|---|---|
| | | | | |
| FEV1 | 2.58 | | 3.42 | l |
| FEV1% | 92.8 | | 123.1 | % |
| FVC | 4.14 | | 4.38 | l |
| TLC | 7.31 | | 9.34 | l |
| RV | 3.14 | | 2.41 | |
| DLCO | 7.3 | | 9.34 | |
| Bode Index | 1 | | | |
| | | | | |

| **Cardiac function** | | | | |
|---|---|---|---|---|
| | | | | |
| **ECG** | | | | |
| PQ | | | 160 | |
| QRS | 105 | | 80 | |
| QT | 340 | | 360 | |

### Description:

57 year-old male patient who is known to suffer from asthma. The FEV 1 % and arterial oxygen saturation are normal. The x-ray done on day 1 suspects a congestion and cardiomegaly, but is not confirmed in the biomarker concentrations. In a computer tomography scan done at timepoint 3, an enlargement of the left atrium of the heart was found (left atrial enlargement LAE), which would be consistent with the slightly increased NT-proBNP value of 238 pg/mL. The chest x-ray done at the same dome does not indicate any congestion or pulmonary edema which would be consistent with the KL-6 value within normal range. An ECG, however, shows that the patient suffered from cardiac arrhythmia, which possibly has contributed to his dyspnea. C-proSP-B values are within normal values as well indicating that the dyspnea the patient suffered from is not caused by an injury to the alveolar membrane layer. The marker combination C-proSP-B, KL-6 and NT-proBNP may therefore be an aid in the differential diagnosis of patients with dyspnea and help to rule out potential causes as well as an aid in the monitoring of patients and the early recognition of changes within the cardiopulmonary system.

## Claims

1. A method for differentiating in a patient suffering from acute shortness of breath between
(a) a pulmonary disease with (a1) pulmonary edema or without (a2) pulmonary edema,
(b) a cardiovascular complication with (b1) pulmonary edema and/or congestion, or without (b2) pulmonary edema and/or congestion,
(c) a cardiovascular complication accompanied by a pulmonary disease with (c1) pulmonary edema and/or congestion, or without (c2) pulmonary edema and/or congestion, or
(d) acute dyspnea without cardiovascular or pulmonary causes,
said method comprising the steps of:
i) measuring the level of C-terminal proSP-B or proSP-B in a sample of said patient,
ii) measuring the level of a BNP-type peptide in a sample of said subject,
iii) measuring the level of a KL-6 in a sample of said subject, and
iv) differentiating between (a1), (a2), (b1), (b2), (c1), (c2) and (d) by comparing the levels determined in i), ii) and iii) with reference levels.

2. The method of claim 1, wherein the sample is obtained at presentation at a physician or at the emergency room or up to ten days after presentation.

3. The method of claims 1 and 2, wherein the biomarker measured in step i) is C-terminal proSP-B, and/or wherein the BNP-type peptide measured in step ii) is NT-proBNP.

4. The method of any one of claims 1 to 3, wherein the pulmonary disease in (c) is caused by the cardiovascular complication, or wherein the cardiovascular complication in (c) is caused by the pulmonary disease, or wherein the pulmonary disease in (c) is independent of the cardiovascular complication.

5. The method of any one of claims 1 to 4, wherein
(a1) a level of C-terminal proSP-B or proSP-B of larger than the reference level for said C-terminal proSP-B or proSP-B, a level of a BNP-type peptide of lower than the reference level of said BNP-type peptide, and a level of KL-6 of larger than the reference level for said KL-6 is indicative for a pulmonary disease with pulmonary edema,
(a2) a level of C-terminal proSP-B or proSP-B of larger than the reference level for said C-terminal proSP-B or proSP-B, a level of a BNP-type peptide of lower than the reference level of said BNP-type peptide, and a level of KL-6 of lower than the reference level for said KL-6 is indicative for a pulmonary disease without pulmonary edema,
(b1) a level of C-terminal proSP-B or proSP-B of lower than the reference level for said C-terminal proSP-B or proSP-B, a level of a BNP-type peptide of larger than the reference level of said BNP-type peptide, and a level of KL-6 of larger than the reference level for said KL-6 is indicative for a cardiovascular complication with pulmonary edema and/or congestion,
(b2) a level of C-terminal proSP-B or proSP-B of lower than the reference level for said C-terminal proSP-B or proSP-B, a level of a BNP-type peptide of larger than the reference level of said BNP-type peptide, and a level of KL-6 of lower than the reference level for said KL-6 is indicative for a cardiovascular complication without pulmonary edema and/or congestion,
(c1) a level of C-terminal proSP-B or proSP-B of larger than the reference level for said C-terminal proSP-B or proSP-B, a level of a BNP-type peptide of larger than the reference level of said BNP-type peptide, and a level of KL-6 of larger than the reference level for said KL-6 is indicative for a cardiovascular complication accompanied by a pulmonary disease with pulmonary edema and/or congestion,
(c2) a level of C-terminal proSP-B or proSP-B of larger than the reference level for said C-terminal proSP-B or proSP-B, a level of a BNP-type peptide of larger than the reference level of said BNP-type peptide, and a level of KL-6 of lower than the reference level for said KL-6 is indicative for a cardiovascular complication accompanied by a pulmonary disease without pulmonary edema and/or congestion, and/or
(d) a level of C-terminal proSP-B or proSP-B of lower than the reference level for said C-terminal proSP-B or proSP-B and a level of a BNP-type peptide of lower than the reference level of said BNP-type peptide is indicative for acute dyspnea without cardiovascular or pulmonary causes.

6. A method for monitoring a patient suffering from acute shortness of breath, said method comprising
(a) measuring the level of C-terminal proSP-B or proSP-B in a first and second sample of said patient,
(b) measuring the level of a BNP-type peptide in a first and second sample of said subject,
(c) measuring the level of a KL-6 in a first and second sample of said subject, and
(d) comparing the level of the biomarker in the second sample as measured in the steps i), ii) and iii), respectively, to the level of said biomarker in said first sample as measured in steps i), ii), and iii), respectively.

7. The method of claim 6, wherein the second sample has been obtained after the first sample, in particular wherein the second sample has been obtained not more than 10 days of after the first sample.

8. The method of claims 6 or 7, wherein an increase of the level of C-terminal proSP-B or proSP-B in the second sample as compared to the first sample as measured in step i) is indicative for a deterioration of pulmonary disease, whereas a decrease of C-terminal proSP-B or proSP-B in the second sample as compared to the first sample is indicative for an improvement of pulmonary disease, and/or
wherein an increase of the level of the BNP-type peptide in the second sample as compared to the first sample as measured in step ii) is indicative for a deterioration of a cardiovascular complication, whereas a decrease of the level of said BNP-type peptide in the second sample as compared to the first sample is indicative for an improvement of a cardiovascular complication, and/or
wherein an increase of the level of KL-6 in the second sample as compared to the first sample as measured in step iii) is indicative for a deterioration of pulmonary edema and/or congestion, whereas a decrease of the level of said BNP-type peptide in the second sample as compared to the first sample is indicative for an improvement of pulmonary edema and/or congestion.

9. A method for diagnosing whether a patient who suffers from a acute shortness of breadth has pulmonary edema and/or congestion, said method comprising the steps:
(a) measuring the level of a KL-6 in a sample of said patient,
(b) comparing the level of KL-6 to a reference level, and optionally,
(c) providing a diagnosis whether the patent has pulmonary edema and/or congestion.

10. The method of claims 9, wherein a level of KL-6 above the reference level is indicative for a patient who has pulmonary edema and/or congestion, and/or wherein a level of KL-6 below the reference level is indicative for a patient who does not have pulmonary edema and congestion.

11. The method of any one of claims 1 to 10, wherein the patient requires mechanical ventilation.

12. The method of any one of claims 1 to 11, wherein the sample is a blood, blood serum or blood plasma sample.

13. Use of
x) C-terminal proSP-B or proSP-B,
y) a BNP-type peptide and
z) KL-6
and/or of
x1) a binding agent which specifically binds to C-terminal proSP-B or proSP-B,
y1) a binding agent which specifically binds to a BNP-type peptide, and
z1) a binding agent which specifically binds to KL-6
in a sample of a patient who suffers from acute shortness of breath for differentiating between
(a) a pulmonary disease with (a1) pulmonary edema or without (a2) pulmonary edema,
(b) a cardiovascular complication with (b1) pulmonary edema and/or congestion, or without (b2) pulmonary edema and/or congestion,
(c) a cardiovascular complication accompanied by a pulmonary disease with (c1) pulmonary edema and/or congestion, or without (c2) pulmonary edema and/or congestion, or
(d) acute dyspnea without cardiovascular or pulmonary causes.

14. A kit comprising a) a binding agent which specifically binds to C-terminal proSP-B or proSP-B, b) a binding agent which specifically binds to a BNP-type peptide, and c) a binding agent which specifically binds to KL-6.

15. A device adapted for carrying out a method according to any one of claims 1 to 5 and 11 and 12, said device comprising
i) an analyzer unit comprising a binding agent which specifically binds to C-terminal proSP-B or proSP-B, a binding agent which specifically binds to a BNP-type peptide, and a binding agent which specifically binds to KL-6, said unit being adapted for measuring the levels of the biomarkers in a sample of a patient having acute shortness of breath, and
ii) an analyzer unit for comparing the determined levels with reference levels, whereby it is differentiated between
(a) a pulmonary disease with (a1) pulmonary edema or without (a2) pulmonary edema,
(b) a cardiovascular complication with (b1) pulmonary edema and/or congestion, or without (b2) pulmonary edema and/or congestion,
(c) a cardiovascular complication accompanied by a pulmonary disease with (c1) pulmonary edema and/or congestion, or without (c2) pulmonary edema and/or congestion, or
(d) acute dyspnea without cardiovascular or pulmonary causes,
said unit comprising a database with reference levels and a computer-implemented algorithm for carrying out the comparison.
